# EUROPÄISCHE PATENTSCHRIFT

(11) **EP 2 982 392 B1**
(45) Veröffentlichungstag und Bekanntmachung des Hinweises auf die Patenterteilung: **28.09.2022**
(21) Anmeldenummer: 15175398.5
(22) Anmeldetag: 06.07.2015
(51) Int. Cl.: A61L 33/00, A61L 31/02, A61L 31/16, A61L 31/08

(54) **MEDIZINISCHE VORRICHTUNG ZUR BEHANDLUNG VON NEUROVASKULÄREN ERKRANKUNGEN SOWIE SYSTEM UND SET MIT EINER DERARTIGEN VORRICHTUNG**
MEDICAL DEVICE FOR TREATING NEUROVASCULAR DISEASES AND SYSTEM AND SET WITH SUCH A DEVICE
DISPOSITIF MEDICAL DESTINE AU TRAITEMENT DE MALADIES NEUROVASCULAIRES ET SYSTEME ET KIT PRESENTANT UN TEL DISPOSITIF

(30) Priorität: 05.08.2014 DE 102014111117
(43) Veröffentlichungstag der Anmeldung: 10.02.2016
(73) Patentinhaber: Acandis GmbH, 75177 Pforzheim (DE)
(72) Erfinder: Cattaneo, Giorgio, 76199 Karlsruhe (DE); Wendel, Hans-Peter, 72336 Balingen (DE)
(74) Vertreter: Kilchert, Jochen

(56) Entgegenhaltungen:
- EP-A1- 1 173 110
- EP-B1- 1 173 110
- WO-A2-02/34163
- US-B1- 6 264 689

## Beschreibung

Die Erfindung betrifft eine Vorrichtung zur Behandlung von neurovaskulären Erkrankungen sowie ein System und ein Set mit einer solchen Vorrichtung.

Zur Behandlung von neurovaskulären Erkrankungen, insbesondere Stenosen, Aneurysmen oder Plaque-Ablagerungen werden vermehrt medizinische Vorrichtungen eingesetzt, die eine selbstexpandierbare Netzstruktur aufweisen. Derartige Vorrichtungen können als Flow-Diverter oder Stents ausgebildet sein, wobei Stents insbesondere als Coil-Schutz oder zur Ausweitung von Stenosen bzw. Offenhalten von Gefäßen eingesetzt werden. Die Nutzung solcher medizinischen Vorrichtungen in intracraniellen Gefäßen ist aufgrund der Miniaturisierungsbemühungen der letzten Jahre ermöglicht worden. Schwierigkeiten, die sich aufgrund der relativ kleinen Gefäßdurchmesser ergeben, wurden in vielen Fällen überwunden.

Problematisch zeigt sich jedoch weiterhin, dass Stents oder ähnliche Implantate im implantierten Zustand eine erhöhte Neigung zur Thrombenbildung bewirken. Langfristig sind daher Folgeerkrankungen, beispielsweise einen durch einen das Gefäß verschließenden Thrombus ausgelösten Schlaganfall, zu befürchten.

Um derartige Folgeerkrankungen zu vermeiden, werden den Patienten systemisch Medikamente verabreicht, die die Blutgerinnung hemmen. Derartige Antikoagulanzien wirken der Gerinnungskaskade entgegen und reduzieren so die Thrombenbildung. Gleichzeitig wird jedoch auch die Blutungsneigung erhöht. Ferner bestehen eine Reihe von Nebenwirkungen, die zu weiteren Beeinträchtigungen bei den Patienten führen können. Außerdem geht mit der notwendigen, medikamentösen Dauerbehandlung die Gefahr einher, dass Patienten die Medikamenteneinnahme vergessen, so dass die gerinnungshemmende Wirkung nicht einsetzt. Es besteht daher ein generelles Bedürfnis nach neurovaskulären Implantaten, die derart ausgebildet sind, dass eine begleitende, systemische Medikamentenbehandlung vermieden oder reduziert werden kann. Insbesondere könnte die sogenannte antithrombozytäre Behandlung bei Verzicht auf ein Medikament auf das zweite Medikament beschränkt werden, wodurch die Risiken erheblich verringert werden könnten.

WO 00/62711 A1 offenbart einen Stent, der aus einem helixförmig um eine Längsachse gewundenem Band gebildet ist. Diese Gestaltung des Stents ermöglicht zwar die Zuführung des Bands über einen sehr kleinen Katheter, wobei das Band im längsgestreckten Zustand durch den Katheter geführt wird. Im helixförmig aufgeweiteten Zustand weist der bekannte Stent allerdings eine geringe Stützwirkung auf, da stabilisierende Querverstrebungen zwischen den einzelnen Helixwindungen des Bandes fehlen.

WO 02/34163 A2 beschreibt einen Stent zur Behandlung von neurovaskulären Erkrankungen, der aus mehreren mäanderförmig angeordneten Stegen gebildet ist. Die mäanderförmig ausgerichteten Stege bilden Stentsegmente, die über Verbinder miteinander gekoppelt sind. Die mäanderförmig angeordneten Stege bilden Spitzen, wobei nur eine begrenzte Zahl gegenüberliegender Spitzen durch Verbinder miteinander gekoppelt sind. Die Mehrzahl der Spitzen liegt frei. Diese freiliegenden Spitzen können sich bei der Zuführung des Stents im Katheter oder im Gewebe verhaken und bieten insoweit ein Verletzungsrisiko. Insbesondere ist ein Zurückziehen des Stents in den Katheter nach einer Teilentlassung kaum möglich, da die sich bereits radial nach außen aufgestellten Spitzen an der Katheteröffnung verhaken würden. Eine Fehlpositionierung des Stents ist daher nicht korrigierbar.

Die Aufgabe der Erfindung besteht darin, eine medizinische Vorrichtung anzugeben, die sich einerseits gut in neurovaskuläre Gefäße einführen lässt und andererseits eine begleitende Einnahme von Antikoagulanzien vermeidet oder reduziert. Ferner besteht die Aufgabe der Erfindung darin, ein System sowie ein Set mit einer solchen Vorrichtung anzugeben.

Erfindungsgemäß wird diese Aufgabe im Hinblick auf die medizinische Vorrichtung durch den Gegenstand des Patentanspruchs 1, im Hinblick auf das System durch den Gegenstand des Patentanspruchs 4 und im Hinblick auf Set durch den Gegenstand des Patentanspruchs 5 gelöst.

So beruht die Erfindung auf dem Gedanken, eine medizinische Vorrichtung zur Behandlung von neurovaskulären Erkrankungen anzugeben, die eine selbstexpandierbare Netzstruktur aufweist. Die selbstexpandierbare Netzstruktur bildet zumindest abschnittsweise eine gekrümmte Wandung und weist in einem radial komprimierten Zustand einen Querschnittsdurchmesser auf, der höchstens 1 mm beträgt. Dabei ist die Netzstruktur aus wenigstens einem Netzstrukturelement gebildet, das eine entlang eines Durchmessers der Netzstruktur messbare Höhe aufweist, die höchstens 75 µm beträgt. Erfindungsgemäß umfasst das Netzstrukturelement eine antithrombogene Beschichtung.

Indem die Netzstruktur im radial komprimierten Zustand einen relativ kleinen Querschnittsdurchmesser aufweist, ist es möglich, die medizinische Vorrichtung zur Behandlung von neurovaskulären Erkrankungen einzusetzen. Insbesondere kann die medizinische Vorrichtung gut in Blutgefäße des neurovaskulären Gefäßsystems eingeführt werden. Die Höhe des Netzstrukturelements, die entlang eines Durchmessers durch die Netzstruktur ermittelt wird, ist ebenfalls vergleichsweise klein, so dass im implantierten Zustand der medizinischen Vorrichtung ein ausreichender Blutfluss durch das neurovaskuläre Gefäß sichergestellt ist. Insgesamt ist die erfindungsgemäße medizinische Vorrichtung also für den Einsatz in neurovaskulären Blutgefäßen angepasst bzw. dimensioniert. Gleichzeitig weist das Netzstrukturelement, das die Netzstruktur der medizinischen Vorrichtung bildet, eine antithrombogene Beschichtung auf.

Die gekrümmte Wandung ist im komprimierten Zustand durch die Innenwand des Katheters bestimmt. Im expandierten Zustand im Gefäß ist die Wandung ebenfalls gekrümmt. Bspw. kann es sich bei der gekrümmten Wandung um eine rohrförmige und/oder trichterförmige Wandung handeln. Andere im Zusammenhang mit endovaskulären Implantaten bekannte Wandungsformen sind möglich. Vorzugsweise ist die Vorrichtung ein Stent oder Flowdiverter.
Die Beschichtung weist antithrombogene Eigenschaften auf. Es ist auch möglich, dass die Beschichtung eine Kombination aus antithrombogenen Eigenschaften sowie aus Hyperplasie verhindernden und/oder Endothelialisierung fördernden Eigenschaften aufweist.

Konkret kann die Beschichtung eine antikoagulierende Wirkung entfalten, so dass die Anhaftung von Blutproteinen günstig beeinflusst wird. Somit kann sich über die Oberfläche des Netzstrukturelements bzw. der Netzstruktur eine Schicht aus Zellen, insbesondere Endothelzellen, bilden. Das eigentliche künstliche Material der Netzstruktur wird somit durch eine Blutproteinschicht maskiert, die zudem die Ablagerung von Thrombozyten und Gerinnungsproteinen, beispielsweise Fibrinogen, verhindert bzw. reduziert, welche für die Bildung von Thromben hauptsächlich verantwortlich sind. Insgesamt ergibt sich somit eine Reduktion der Thrombenbildungsneigung. Ursächlich dafür ist die antithrombogene bzw. Thrombozyten-Aggregations-hemmende Beschichtung.

Im Allgemeinen kann die Beschichtung sich über die gesamte Netzstruktur erstrecken. Es ist auch möglich, dass die Netzstruktur bzw. das Netzstrukturelement teilweise mit der Beschichtung versehen ist. Ferner ist es denkbar, dass die Beschichtung, insbesondere die antithrombogene und/oder die Endothelialisierung fördernde Beschichtung hauptsächlich an einer Innenumfangsfläche der Netzstruktur angeordnet ist. Jedenfalls ist vorgesehen, dass diejenigen Flächen der Netzstruktur mit einer Beschichtung versehen sind, die im implantierten Zustand mit Blut in Kontakt gelangen. Die Beschichtung kann nur an der Außenfläche angeordnet sein, z.B. im Fall einer Hyperplasie verhindernden Beschichtung. Eine erste Beschichtung, insbesondere die antithrombogene und/oder die Endothelialisierung fördernde Beschichtung, kann an der Innenfläche anhaften und eine zweite Beschichtung, insbesondere die Hyperplasie verhindernden Beschichtung, kann an der Außenfläche anhaften.

Es ist auch möglich, dass mehrere Beschichtungen in der Form mehrerer aufeinander angeordneter Schichten an derselben Fläche, insbesondere der Innenfläche und/oder Außenfläche, anhaften. Dabei haftet eine erste Schicht direkt an der Fläche an. Die nächste Schicht ist auf die erste Schicht aufgebracht.

Weitere mögliche Schichten sind entsprechend aufeinander angeordnet. Bspw. kann sich die antithrombogene Beschichtung unter der die Endothelialisierung fördernden Beschichtung angeordnet sein. Auch hier sind alle Kombinationen möglich.

Die medizinische Vorrichtung, insbesondere die Netzstruktur, kann kleinere Dimensionen als oben angegeben aufweisen. Beispielsweise kann die Netzstruktur im radial komprimierten Zustand einen Querschnittsdurchmesser aufweisen, der höchstens 0,7 mm, insbesondere höchstens 0,5 mm, insbesondere höchstens 0,4 mm, beträgt. Die Höhe des Netzstrukturelements, gemessen entlang des Durchmessers der Netzstruktur, kann kleiner als 75 µm sein, beispielsweise höchstens 70 µm, insbesondere höchstens 60 µm, vorzugsweise 50 µm.

Bei einer bevorzugten Ausführungsform der medizinischen Vorrichtung weist die Beschichtung eine Schichtdicke zwischen 5 nm und 50 nm auf. Insbesondere kann die antithrombogene Beschichtung eine Schichtdicke zwischen 10 nm und 30 nm, vorzugsweise zwischen 15 nm und 25 m, im Speziellen 20 nm aufweisen. Im Erfindungsgemäß beträgt die Schichtdicke der Beschichtung zwischen 5 nm und 100 nm. Die Schichtdicke der antithrombogenen Beschichtung kann beispielsweise über ein Raster-Kraft-Mikroskop ermittelt werden (atomic force microscopy / AFM-Messmethode).

Erfindungsgemäß ist die Beschichtung mehrschichtig ausgebildet. Dabei umfasst wenigstens eine erste Schicht Albumin oder Heparin und eine zweite Schicht einen Haftvermittler (Primer). Die zweite Schicht ist zwischen der Netzstruktur und der ersten Schicht angeordnet. Durch den Haftvermittler haftet Albumin gut an dem Material der Netzstruktur, das beispielsweise durch eine Nickel-TitanLegierung gebildet sein kann. Als Haftvermittler eignet sich 1-Ethyl-3-Carbodiimid-Hydrochlorid (EDC). Das EDC kann zusätzlich N-Hydroxysuccinimid aufweisen (EDC-NHS).

Vorzugsweise ist die Beschichtung derart langzeitbeständig bzw. stabil, dass die Masse der Beschichtung bei einem Kontakt mit Blut oder einer physiologischen Ersatzflüssigkeit, insbesondere mit einer Natriumchlorid-Lösung oder einer Ringer-Lactat-Lösung, über einen Zeitraum von wenigstens vier Stunden, insbesondere wenigstens 30 Tagen, um höchstens 5%, insbesondere um höchstens 3%, insbesondere um höchstens 1%, reduziert wird. Damit ist sichergestellt, dass die Wirkung der Beschichtung über einen ausreichend langen Zeitraum besteht.

Besonders bevorzugt ist es, wenn die Beschichtung derart langzeitbeständig bzw. stabil ist, dass die Masse der Beschichtung bei einem Kontakt mit Blut oder einer physiologischen Ersatzflüssigkeit, insbesondere mit einer Natriumchlorid-Lösung oder eienr Ringer-Lactat-Lösung, über einen Zeitraum von wenigstens vier Stunden, insbesondere wenigstens 30 Tagen, vollständig erhalten bleibt. Ein solcher Zeitraum ermöglicht es beispielsweise, dass die medizinische Vorrichtung von einer Endothelzellenschicht überzogen wird, so dass eine Thrombenbildung auf natürliche Weise vermieden wird. Die antithrombogene Beschichtung überbrückt insofern den Zeitraum bis zur natürlichen Einheilung bzw. Einkapselung der medizinischen Vorrichtung in eine Neointimaschicht, insbesondere aus Endothelzellen, die sich um die Netzstrukturelemente bildet.

Die Nutzung einer physiologischen Ersatzflüssigkeit zum Test der Langzeitbeständigkeit der Beschichtung ermöglicht einen objektiven Vergleich. Ferner wird durch die Verwendung der Ersatzflüssigkeit, die dem menschlichen Blut vorzugsweise ähnlich ist, erreicht, dass daraus objektive Erfahrungswerte ermittelt werden können, die auf das Verhalten der Beschichtung im implantierten Zustand schließen lassen, wenn die Beschichtung dem menschlichen Blutfluss ausgesetzt ist. Daher werden als Ersatzflüssigkeiten vorzugsweise eine 0,9-prozentige Natriumchlorid-Lösung oder eine Ringer-Lactat-Lösung verwendet. Derartige Ersatzflüssigkeiten sind isotonisch und eignen sich gut als Indikator für das Verhalten der Beschichtung im implantierten Zustand.

Um zu vermeiden, dass die Beschichtung bei der Zuführung der medizinischen Vorrichtung durch einen Katheter abgerieben wird, ist die Beschichtung vorzugsweise abriebfest. Insbesondere kann die Beschichtung derart abriebfest sein, dass die Masse der Beschichtung beim einmaligen Durchschieben der Netzstruktur durch einen Katheter mit einer Länge von 155 cm bis 165 cm um höchstens 30%, insbesondere um höchstens 20%, insbesondere um höchstens 10%, insbesondere um höchstens 5%, reduziert wird. Die Beschichtung kann auch derart abriebfest sein, dass die Masse der Beschichtung beim einmaligen Durchschieben der Netzstruktur durch einen Katheter mit einer Länge von 155 cm bis 165 cm vollständig erhalten bleibt. Die verbesserte Abriebfestigkeit der Beschichtung ermöglicht es, die medizinische Vorrichtung direkt durch einen Katheter an den Behandlungsort zu führen. Insbesondere kann auf eine sich zwischen der Katheterinnenwand und der medizinischen Vorrichtung, insbesondere der Netzstruktur, erstreckende Schutzumhüllung verzichtet werden. Dies reduziert insgesamt die Dimensionen des gesamten Katheter-Sets mit der medizinischen Vorrichtung, so dass kleinere Blutgefäße erreicht werden.

Der Katheter, durch den die medizinische Vorrichtung geschoben wird, kann einen Innendurchmesser aufweisen, der höchstens 1 mm, insbesondere höchstens 0,7 mm, insbesondere höchstens 0,5 mm, insbesondere höchstens 0,4 mm, beträgt. Ferner kann der Katheter zumindest abschnittsweise eine Krümmung umfassen, die einen Bogen mit einem Winkel von 180° bildet und deren Krümmungsradius höchstens 5 mm, insbesondere höchstens 3 mm, beträgt. Die Beschichtung ist vorzugsweise derart abriebfest, dass sie auch beim einmaligen Schieben durch einen Katheter, der die vorgenannte Krümmung und den vorgenannten Innendurchmesser aufweist, vollständig erhalten bleibt, jedenfalls höchstens 20%, insbesondere höchstens 10%, insbesondere höchstens 5%, ihrer Masse verliert. Damit ist sichergestellt, dass die Beschichtung auch dann zumindest weitgehend erhalten bleibt, wenn die medizinische Vorrichtung bzw. die Netzstruktur durch starke Gefäßkrümmungen an den Behandlungsort geschoben werden muss. Diese Werte gelten für eine vollständige Beschichtung der Stege, d.h. sowohl an der Innen- als auch Außenfläche.

Im expandierten Zustand kann die Netzstruktur einen Querschnittsdurchmesser von höchstens 6,5 mm, insbesondere höchstens 6 mm, insbesondere höchstens 5,5 mm, insbesondere höchstens 5 mm, insbesondere höchstens 4,5 mm, insbesondere höchstens 3,5 mm, insbesondere höchstens 3 mm, insbesondere höchstens 2,5 mm aufweisen. Dabei entspricht der expandierte Zustand dem kraftunbelasteten, vollständig aufgeweiteten Zustand der Netzstruktur. Insgesamt ist vorgesehen, dass die Netzstruktur einen hohen Expansionsgrad aufweist, d.h. das Verhältnis zwischen dem Querschnittsdurchmesser der Netzstruktur im komprimierten Zustand und dem Querschnittsdurchmesser der Netzstruktur im expandierten Zustand ist klein, insbesondere kleiner als 1/5, vorzugsweise kleiner als 1/8, beispielsweise kleiner als 1/10.

Bei einer bevorzugten Ausführungsform der Erfindung ist die Netzstruktur durch eine Vielzahl von einstückig miteinander verbundenen Stegen gebildet. Dabei bildet jeder Steg jeweils ein Netzstrukturelement. Die messbare Höhe des Netzstrukturelements entspricht dabei der Steghöhe. Derartige Gitterstrukturen können beispielsweise durch Laserschneiden aus einem Vollmaterial, insbesondere aus einem Rohr oder einem Draht, oder durch ein Gasphasen-Abscheideverfahren, beispielsweise ein PVD- oder CVD-Verfahren, hergestellt werden. Vorzugsweise werden derartige Netzstrukturen durch ein Sputter-Verfahren hergestellt.

Alternativ kann die Netzstruktur durch ein Drahtgeflecht aus einem einzigen Draht oder mehreren Drähten gebildet sein. Der Draht oder die mehreren Drähte bilden jeweils ein Netzstrukturelement, wobei die messbare Höhe des Netzstrukturelements wegen der Überkreuzung der Drähte dem doppelten Drahtdurchmesser entspricht. Im Allgemeinen kann das Drahtgeflecht der Netzstruktur aus einem einzigen Draht oder mehreren Drähten gebildet sein. Bei der Bildung des Drahtgeflechts aus einem einzigen Draht ist vorgesehen, dass der einzige Draht an wenigstens einem Längsende der Netzstruktur umgelenkt und zurückgeführt wird. Durch mehrfache Umlenkung und Zurückführung des einzigen Drahts ergibt sich ein Drahtgeflecht. Die freien Enden des Drahts können an einem Längsende der Netzstruktur offenbleiben oder an beliebiger Stelle entlang der Netzstruktur miteinander verbunden werden. Bei der Bildung eines Drahtgeflechts aus mehreren Drähten können diese an den Längsenden der Netzstruktur offen sein. Es ist auch möglich, dass die Netzstruktur an einem Längsende geschlossene Geflechtenden aufweist, die durch Umlenken der einzelnen Drähte gebildet sind. An einem gegenüberliegenden Längsende können die Drähte offene Drahtenden aufweisen oder miteinander verbunden sein.

Ein oder mehrere Netzstrukturelemente, insbesondere ein oder mehrere Drähte, können einen Kern aus einem röntgensichtbaren Material und eine Umhüllung aus einem Formgedächtnismaterial aufweisen. Geeignete röntgensichtbare Materialien umfassen beispielsweise Platin oder Platin-Legierungen. Als Formgedächtnismaterialien kommen bevorzugt Nickel-Titan-Legierungen zum Einsatz, beispielsweise Nitinol.

Gemäß einem nebengeordneten Aspekt beruht die Erfindung auf dem Gedanken, ein System mit einer zuvor beschriebenen medizinischen Vorrichtung und einem Transportdraht anzugeben, wobei die medizinische Vorrichtung auf dem Transportdraht angeordnet, insbesondere auf dem Transportdraht lösbar fixiert, ist. Vorzugsweise ist die Netzstruktur der medizinischen Vorrichtung im komprimierten Zustand lösbar mit dem Transportdraht verbunden, der sich in Längsrichtung durch die Netzstruktur erstreckt. Die medizinische Vorrichtung ist vorzugsweise an einer distalen Spitze des Transportdrahtes angeordnet.

Die komprimierte Netzstruktur ist ferner mit dem Transportdraht längsverschieblich innerhalb eines Zuführschlauchs (Introducer) angeordnet. Der Zuführschlauch bzw. Introducer weist vorzugsweise eine Länge zwischen 25 cm und 60 cm auf. Die medizinische Vorrichtung wird somit vorzugsweise vormontiert auf einem Transportdraht und innerhalb eines Zuführschlauchs bzw. Introducers bereitgestellt. Der Innendurchmesser des Zuführschlauchs entspricht vorzugsweise dem Innendurchmesser eines entsprechenden Katheters, der für die Führung der medizinischen Vorrichtung zum Behandlungsort verwendet wird. Das im Introducer auf dem Transportdraht vormontierte Netzstrukturelement kann so einfach von dem Introducer an den Katheter übergeben werden. Insofern ist es vorteilhaft, wenn das erfindungsgemäße System mit der medizinischen Vorrichtung, dem Transportdraht und dem Zuführschlauch als Set mit einem Katheter bereitgestellt wird, wobei der Katheter und der Zuführschlauch gleiche Innendurchmesser aufweisen. Hinsichtlich des Zuführschlauchs ist im Allgemeinen vorgesehen, dass dieser aus PTFE oder FEP gebildet ist.

Ferner wird im Rahmen eines nebengeordneten Aspekts der Erfindung ein Set mit einem Katheter und einer zuvor beschriebenen medizinischen Vorrichtung oder einem zuvor beschriebenen System offenbart und beansprucht, wobei der Katheter einen Katheterschlauch aufweist, dessen Innendurchmesser höchstens 0,7 mm, insbesondere höchstens 0,5 mm, insbesondere höchstens 0,4 mm, beträgt. Dies entspricht nach der in der Medizin üblicherweise verwendeten Einheit Zoll etwa einem Innendurchmesser von vorzugsweise höchstens 0,027 Zoll, insbesondere höchstens 0,021 Zoll, insbesondere höchstens 0,017 Zoll. Mit anderen Worten umfasst das Set vorzugsweise einen Katheter mit einer Größe von 3 French, insbesondere 2,5 French, insbesondere 2 French. Dies betrifft den Außendurchmesser des Katheters. Die vorgenannten Werte können Maximalwerte bilden. Kleinere Kathetergrößen sind möglich.

Der Katheterschlauch kann eine Länge aufweisen, die zwischen 130 cm und 170 cm, insbesondere zwischen 155 cm und 165 cm, insbesondere 160 cm, beträgt. Eine solche Katheterlänge erlaubt es, den Katheter gut über periphere Blutgefäße in das neurovaskuläre Gefäßsystem einzuführen. Somit gelangt die Katheterspitze sicher an den Behandlungsort. Der Transportdraht kann längsverschieblich innerhalb des Katheterschlauchs angeordnet sein und die medizinische Vorrichtung tragen. Insbesondere kann die medizinische Vorrichtung an der distalen Spitze des Transportdrahts angebracht sein, wobei der Transportdraht mit der medizinischen Vorrichtung durch den Katheterschlauch an den Behandlungsort geführt werden kann. In diesem Fall ist die Netzstruktur der medizinischen Vorrichtung vorzugsweise komprimiert, wobei ein verbleibendes Innenlumen der Netzstruktur durch den Transportdraht ausgefüllt wird. Mit anderen Worten beträgt der Außendurchmesser der auf den Transportdraht komprimierten Netzstruktur vorzugsweise dem Durchmesser des Transportdrahts zuzüglich der doppelten Wandstärke der Netzstruktur bzw. der doppelten Höhe des Netzstrukturelements, wenn die Netzstruktur aus eindeutig miteinander verbundenen Netzstrukturelementen oder Stegen gebildet ist. Bei einer Netzstruktur aus einem Geflecht von Drähten, die sich überkreuzen, beträgt der Außendurchmesser der auf dem Transportdraht komprimierten Netzstruktur vorzugsweise dem Durchmesser des Transportdrahts zuzüglich der vierfachen Drahtstärke. Nach Entlassung aus dem Katheterschlauch kann sich die medizinische Vorrichtung bzw. die Netzstruktur selbsttätig, insbesondere radial, aufweiten und so den expandierten Zustand einnehmen.

Die Erfindung wird im Folgenden anhand anhand eines Ausführungsbeispiels unter Bezugnahme auf die beigefügten, schematischen Zeichnungen näher erläutert. Darin zeigen
- Fig. 1: eine Seitenansicht einer medizinischen Vorrichtung, die als Stent ausgebildet ist;
- Fig. 2a bis c: Detailansichten einer medizinischen Vorrichtung mit einer Netzstruktur gemäß Fig. 1 ohne eine erfindungsgemäße Beschichtung nach Durchführung eines in-vitro-Versuchs, wobei sich eine Ablagerung von Plasmaproteinen zeigt; und
- Fig. 3 bis c: Detailansichten einer erfindungsgemäßen medizinischen Vorrichtung, die eine antithrombogene Beschichtung trägt.

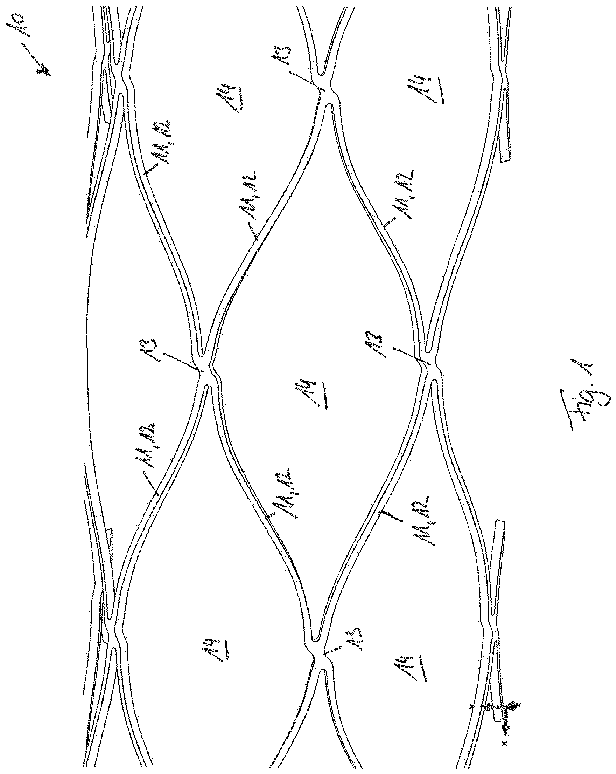

Fig. 1 zeigt in einer Seitenansicht eine medizinische Vorrichtung mit einer Netzstruktur 10, die aus Netzstrukturelementen 11 gebildet ist. Die Netzstrukturelemente 11 sind bei hier dargestellten Ausführungsbeispielen als Stege 12 ausgebildet, die einstückig miteinander verbunden sind, um die Netzstruktur 10 zu bilden. Die Netzstruktur 10 kann folglich aus einem einstückigen Rohmaterial hergestellt sein, beispielsweise durch Laserschneiden eines Rohres oder Drahtes, wobei die Stege 12 freigeschnitten werden. Alternativ kann die Netzstruktur durch ein physikalisches Gasphasen-Abscheideverfahren hergestellt werden, beispielsweise ein Sputter-Verfahren.

Fig. 1 zeigt die Netzstruktur 10 in einem teilexpandierten Zustand. Insbesondere verweist die Netzstruktur 10 gemäß Fig. 1 einen Querschnittsdurchmesser im dargestellten teilexpandierten Zustand auf, der im Wesentlichen zwei Drittel des Querschnittsdurchmessers der Netzstruktur 10 im vollexpandierten Zustand entspricht.

Neben den hier dargestellten Ausführungsbeispielen ist es möglich, die Netzstruktur 10 aus einem Drahtgeflecht zu bilden. Dabei bilden die Drähte des Drahtgeflechts jeweils ein Netzstrukturelement 11. Das Drahtgeflecht kann aus einem einzigen Draht gebildet sein, der an den längsaxialen Enden der Netzstruktur 10 umgelenkt und zurückgeführt ist. Der Draht ist zur Bildung der Netzstruktur 10 mit sich selbst verflochten. Die Netzstruktur 10 kann alternativ auch aus mehreren Drähten gebildet sein, die miteinander verflochten sind. Die mehreren Drähte können an einem längsaxialen Ende jeweils umgelenkt und zurückgeführt sein. An einem gegenüberliegenden längsaxialen Ende können die Drähte offene Drahtenden aufweisen. Es ist auch möglich, dass die miteinander verflochtenen Drähte an beiden längsaxialen Enden offene Drahtenden umfassen.

Insbesondere bei einer medizinischen Vorrichtung, die einen Flow-Diverter bildet, können wenigstens 16, insbesondere wenigstens 24 Drähte die Netzstruktur bilden, wobei die Drähte an wenigstens einem Längsende der Netzstruktur umgelenkt werden. Dadurch entsteht ein Geflecht, das in einem Querschnitt die doppelte Anzahl von Drähten, insbesondere 32 (fiktive) Drähte bzw. 48 (fiktive) Drähte. Im Hinblick auf eine möglichst kleine Porengröße der Netzstruktur ist vorgesehen, dass der Flechtwinkel, also der Winkel zwischen einer in die Wandungsebene der Netzstruktur projizierten Längsachse in de Netzstruktur und einem Draht der Netzstruktur wenigstens 65°, insbesondere wenigstens 75°, beträgt. Bei einem Stent, der durch eine Netzstruktur aus einem einzigen Draht gebildet ist, beträgt der Flechtwinkel vorzugsweise wenigstens 55°, insbesondere wenigstens 60°, insbesondere wenigstens 65°.

Bei der Netzstruktur 10 gemäß den dargestellten Ausführungsformen sind die Stege 12 bzw. Netzstrukturelemente 11 jeweils durch Verbinder 13 miteinander gekoppelt. Dabei treffen sich jeweils vier Stege 12 in einem Verbinder 13. Wie in Fig. 1 gut erkennbar ist, weist der Verbinder 13 einen gekrümmten Verlauf auf, so dass in Umfangsrichtung der Netzstruktur 10 ein Versatz zwischen den Stegen 12 unterschiedlicher Zellen 14 der Netzstruktur 10 besteht. Dies erhöht die (Biege-)Flexibilität der Netzstruktur 10 und erleichtert die Zuführung der Netzstruktur 10 in kleine Blutgefäße, insbesondere im neurovaskulären Bereich.

Die Netzstruktur 10 bildet Zellen 10, die jeweils durch vier Stege 12 begrenzt sind. Insgesamt ergibt sich somit eine im Wesentlichen rautenförmige Grundgeometrie der Zellen 14. In Fig. 1 ist außerdem erkennbar, dass die Netzstruktur 10 Stege 12 mit unterschiedlicher Stegbreite aufweist. Insbesondere ist jede Zelle 14 durch zwei Stegpaare begrenzt, wobei jeweils die im Wesentlichen parallel zueinander verlaufenden bzw. sich gegenüberliegenden und nicht direkt miteinander verbundenen Stege 12 ein Stegpaar bilden. Dabei weisen die Stege 12 eines ersten Stegpaares eine kleinere Stegbreite als die Stege 12 eines zweiten Stegpaares auf. Diese Anordnung der Stege 12 mit unterschiedlicher Stegbreite erhöhte die Biegeflexibilität der Netzstruktur 10 und erleichtert somit die Zuführung der medizinischen Vorrichtung in neurovaskuläre Gefäße, insbesondere wenn diese starke Gefäßkrümmungen aufweisen. Die gute Flexibilität verbessert die Apposition an der Gefäßwand und verhindert daher die Entstehung von Staubereichen, die die Thrombenbildung wiederrum fördern. Die gute Flexibilität spielt besonders in Verbindung mit einer antithrombogenen Beschichtung eine Rolle.

Im Allgemeinen ist es auch möglich, dass die Stege 12 der Netzstruktur 10 gleiche Stegbreiten aufweisen. Ferner kann die Netzstruktur 10 Verbinder 13 aufweisen, die derart gestaltet sind, dass zwischen den Stegen 12 unterschiedlicher Zellen 14 kein Versatz vorliegt.

Die Netzstruktur 10 ist vorzugsweise selbstexpandierbar. Bei Freisetzung der Netzstruktur 10 drängt diese also selbsttätig in den expandierten Zustand. Dabei vergrößert sich der Querschnittsdurchmesser der Netzstruktur 10. Insbesondere ist vorgesehen, dass die Netzstruktur 10 von einem komprimierten Zustand in einen expandierten Zustand expandierbar ist, wobei das Verhältnis zwischen dem komprimierten Zustand und expandierten Zustand vorzugsweise 0,1 oder größer beträgt.

In Fig. 1 wird ferner angedeutet, dass die Netzstruktur 10 im Wesentlichen rohrförmig ausgebildet ist bzw. eine rohrförmige Wandung bildet. Die rohrförmige Wandung umfasst eine Wandstärke, die der Steghöhe der Stege 12 entspricht. Die Steghöhe ist entlang eines Durchmessers der Netzstruktur 10 messbar und beträgt vorzugsweise höchstens 75 µm. Weiter bevorzugt ist es, wenn die Steghöhe nicht größer als 70 mm, insbesondere höchstens 60 µm, beträgt. Der Querschnittsdurchmesser der Netzstruktur 10 im radial komprimierten Zustand beträgt höchstens 1 mm, vorzugsweise höchstens 0,7 mm, insbesondere höchstens 0,5 mm, insbesondere höchstens 0,4 mm. Dies stellt sicher, dass die Netzstruktur 10 bzw. die medizinische Vorrichtung gut in kleine Blutgefäße, insbesondere im intrazerebralen Bereich, einführbar ist.

Die Stege 12 der Netzstruktur 10 weisen ferner eine Stegbreite auf, die in Umfangsrichtung der Netzstruktur 10 ermittelt wird. Die Stegbreite beträgt vorzugsweise höchstens 50 µm, insbesondere höchstens 40 µm. In Extremfällen oder bei einer Netzstruktur 10, die Stege 12 mit unterschiedlicher Stegbreite aufweist, können die schmaleren Stege 12 eine Stegbreite von höchstens 35 µm, insbesondere höchstens 32 µm, insbesondere höchstens 30 µm, vorzugsweise höchstens 25 µm, aufweisen. Die Mindest-Stegbreite beträgt vorzugsweise 15 µm.

Im Allgemeinen kann die Netzstruktur 10 für unterschiedliche Arten von medizinischen Vorrichtungen eingesetzt werden. Besonders bevorzugt ist es, wenn die medizinische Vorrichtung einen Stent bildet, insbesondere die Netzstruktur 10 als Stent ausgebildet ist. In diesem Fall ist die Netzstruktur 10 vorzugsweise rotationssymmetrisch ausgebildet bzw. bildet eine im Wesentlichen zylinderförmige Wandung. Alternativ kann vorgesehen sein, dass die medizinische Vorrichtung als Flow-Diverter bzw. als Thrombenfänger ausgebildet ist, wobei die Netzstruktur 10 nur abschnittsweise eine rohrförmige oder rotationssymmetrische Wandung aufweist.

Im Allgemeinen kann vorgesehen sein, dass die medizinische Vorrichtung implantierbar ist. Dies trifft insbesondere für Stents oder Flow-Diverter zu. Wenn die medizinische Vorrichtung einen Thrombenfänger bildet, ist dieser vorzugsweise fest mit einem Transportdraht gekoppelt und wird unmittelbar nach dem Einsatz im Blutgefäß zusammen mit dem Transportdraht entfernt.

In einer besonders bevorzugten Variante bildet die Netzstruktur 10 einen Stent, wobei die Netzstruktur 10 durch Laserschneiden oder ein Sputter-Verfahren hergestellt ist. Derartige Stents weisen vorzugsweise eine Porengröße auf, die im expandierten Zustand der Netzstruktur 10 höchstens 2,5 mm, insbesondere höchstens 2,0 mm, insbesondere höchstens 1,7 mm, insbesondere höchstens 1,3 mm, insbesondere 1,1 mm, beträgt. Die Porengröße wird durch den größtmöglichen Kreis bestimmt, der in eine Zelle 14 einbeschrieben werden kann. Dabei berührt der Kreis die Stege 12 der Zelle 14. Mit anderen Worten entspricht die Porengröße einem maximalen Durchmesser eines zylinderförmigen Stifts, der durch die Zelle 14 im expandierten Zustand der Netzstruktur 10 hindurchgeführt werden kann. Bei geflochtenen Stents beträgt die Porengröße vorzugsweise höchstens 1,2 mm, insbesondere höchstens 0,9 mm.

Ein Stent mit einer lasergeschnittenen oder gesputterten Netzstruktur 10 umfasst vorzugsweise zwischen 3 und 12, insbesondere zwischen 4 und 9, vorzugsweise 6, Zellen entlang des Umfangs der Netzstruktur 10. Bei einem geflochtenen Stent beträgt die Anzahl der Zellen in Umfangsrichtung der Netzstruktur 10 vorzugsweise zwischen 6 und 8, insbesondere 6.

Wie in Fig. 1 gut erkennbar ist, ist zwischen den in Umfangsrichtung benachbarten Stegen 12, die an einem Verbinder 13 miteinander gekoppelt sind, ein Winkel gebildet. Dieser Zellenwinkel beträgt im expandierten Zustand der Netzstruktur 10 vorzugsweise zwischen 70° und 110°, insbesondere 90°. Bei einer Netzstruktur 10, die aus einem Drahtgeflecht gebildet ist, überkreuzen sich die Drähte vorzugsweise unter einem Winkel zwischen 100° und 140°, insbesondere 120°. Dies gilt für den expandierten Zustand der Netzstruktur 10.

Für alle Arten (lasergeschnitten, gesputtert oder geflochten) von Netzstrukturen 10, die als Stent ausgebildet sind, gilt, dass das Verhältnis zwischen dem Querschnittsdurchmesser der Netzstruktur 10 im komprimierten Zustand zum Querschnittsdurchmesser der Netzstruktur 10 im expandierten Zustand vorzugsweise höchstens 1/5, insbesondere höchstens 1/8, vorzugsweise höchstens 1/10 beträgt. Bei lasergeschnittenen oder gesputterten Stents kann dieses Verhältnis kleiner als 0,1 sein, beispielsweise höchstens 0,08 betragen. Eine Netzstruktur 10, die aus einem Drahtgeflecht gebildet ist, kann insbesondere Drähte, oder wenigstens einen Draht aufweisen, der als Verbunddraht ausgebildet ist. Ein derartiger Verbunddraht kann einen Kern aus einem röntgensichtbaren Material und eine den Kern vollständig umhüllende Schicht aus einem Formgedächtnismaterial aufweisen. So wird die Röntgensichtbarkeit der Netzstruktur 10 erhöht, wobei gleichzeitig die selbstexpandierbaren Eigenschaften beibehalten werden.

Wenn die Netzstruktur 10 eine medizinische Vorrichtung in Form eines Flow-Diverters bildet, ist vorgesehen, dass die Porengrößer höchstens 250 µm, insbesondere höchstens 200 µm, insbesondere 150 µm beträgt. Die Anzahl der Zellen in Umfangsrichtung der Netzstruktur 10 beträgt bei Flow-Divertern vorzugsweise zwischen 16 und 48, insbesondere zwischen 20 und 24. Die Stege oder Drähte der Netzstruktur 10 bzw. allgemein die Netzstrukturelemente 11 des Flow-Diverters überkreuzen bzw. treffen sich vorzugsweise unter einem Winkel zwischen 120° und 160°, insbesondere 150°. Bei Flow-Divertern besteht ein vorteilhaftes Verhältnis zwischen dem Durchmesser im komprimierten Zustand der Netzstruktur 10 und dem Durchmesser im expandierten Zustand der Netzstruktur 10 von höchstens 0,12, insbesondere höchstens 0,1, insbesondere höchstens 0,08. Dabei ist es besonders bevorzugt, wenn bei einem Flow-Diverter die Netzstruktur aus einem Drahtgeflecht gebildet ist, das an einem Längsende offene Drahtenden und am gegenüberliegenden Längsende geschlossene Zellen aufweist. Die geschlossenen Zellen werden durch Umlenken der Drähte am längsaxialen Ende der Netzstruktur 10 gebildet.

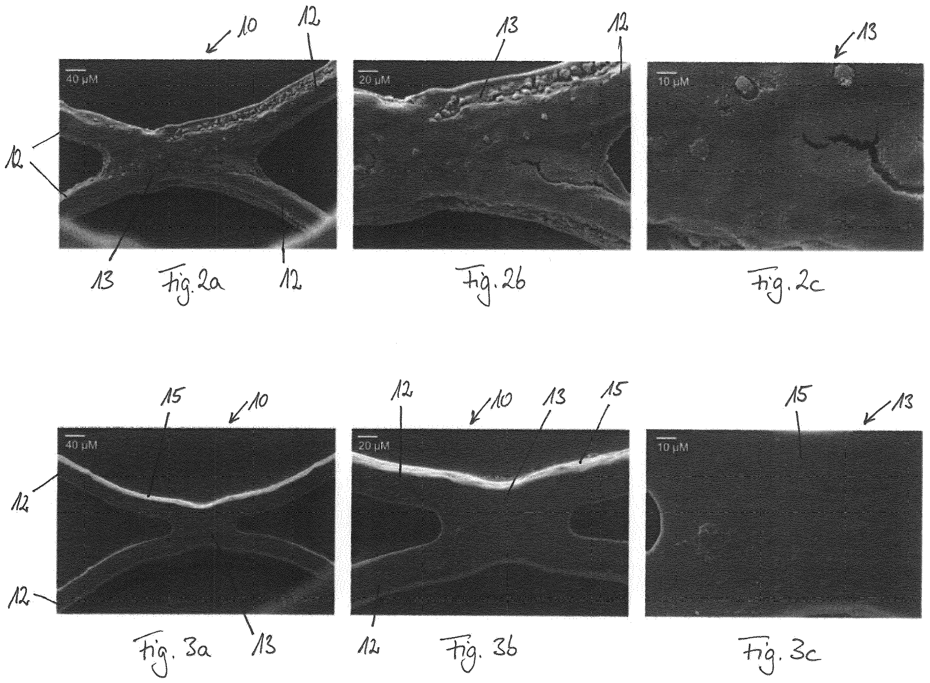

In den Fig. 2a bis 2c ist ein Ausschnitt einer Netzstruktur 10 gezeigt, die keine Beschichtung aufweist. Insbesondere ist ein Verbinder 13 der Netzstruktur 10 dargestellt. Die dargestellte Netzstruktur 10 wurde in einem in-vitro-Versuch für einen Zeitraum von 60 min in einen Schlauch implantiert, der einen Innendurchmesser von 3,2 mm aufweist. Die Höhe des Netzstrukturelements bzw. Stegs 12 beträgt 75 µm. Die Stegbreite liegt bei 35 µm. Im Rahmen des in-vitro-Versuchs wurde menschliches Blut mit einer Temperatur von 37°C und einem Volumenstrom von 150 ml/min stationär durch den Schlauch geleitet, in welchem der Stent bzw. die Netzstruktur 10 implantiert war. Wie gut in den Fig. 2a bis 2c erkennbar ist, hat sich auf der Netzstruktur 10 eine Schicht aus Blutbestandteilen abgelagert, die u.a. Zellen und Plasmaproteine umfasst. Insgesamt zeigt sich, dass bereits nach kurzer Zeit unbehandelte Netzstrukturen 10 mit einer Ablagerung aus Blutbestandteilen "beschichtet" sind, was die Gefahr einer Thrombenbildung erhöht.

In den Fig. 3a bis 3c ist jeweils ein Ausschnitt des Verbinders 13 der Netzstruktur 10 gezeigt, wobei unterschiedliche Vergrößerungen dargestellt sind. Bei diesem erfindungsgemäßen Ausführungsbeispiel ist die Netzstruktur 10 mit einer antithrombogenen Beschichtung 15, versehen, die eine Schichtdicke zwischen 5 nm und 100 nm aufweist und mehrschichtig ausgebildet ist, wobei wenigstens eine erste Schicht Albumin oder Heparin und eine zweite Schicht einen Haftvermittler umfasst, wobei die zweite Schicht zwischen dem Netzstrukturelement 11 und der ersten Schicht angeordnet ist. Im Allgemeinen ist vorgesehen, dass die Beschichtung 15 nicht nur biokompatibel, sondern insbesondere biofunktional ist. Dies bedeutet, dass die Beschichtung 15 vorzugsweise die Endothelialisierung, also die Anlagerung von Endothelzellen, fördert. Die biofunktionale Beschichtung 15 kann Fänger-Moleküle, beispielsweise Aptamere, umfassen. Besonders bevorzugt ist es, wenn die biofunktionale Beschichtung 15 Pegaptanib aufweist, das hochspezifisch ist und mit einer hohen Affinität an den Wachstumsfaktor VEGF (vascular endothelial growth factor) bindet. Ferner kann die biofunktionale Beschichtung 15 Stoffe aufweisen, die eine Hyperplasie reduzieren. Ein derartiger geeigneter Stoff umfasst beispielsweise small interfering RNA (siRNA). Es ist möglich, dass die biofunktionale Beschichtung 15 derartige die Endothelialisierung fördernde oder die Hyperplasie reduzierende Stoffe abgibt und so eine Zellproliferation auf genetischer Ebene verhindert bzw. verzögert.

Erfindungsgemäß umfasst die antithrombogene Beschichtung 15 Albumin und/oder Heparin. Diese antithrombogene Beschichtung 15 ist mehrschichtig wobei wenigstens eine erste Schicht Albumin oder Heparin und eine zweite Schicht einen Haftvermittler aufweist, wobei die zweite Schicht zwischen dem Netzstrukturelement 11 und der ersten Schicht angeordnet ist. Auf der antithrombogenen Beschichtung 15 kann eine die Endothelialisierung fördernde Beschichtung angeordnet sein. Die Schichtdicke der Beschichtung 15 beträgt vorzugsweise zwischen 10 nm und 30 nm, wobei Abweichungen von +/- 2 nm möglich sind. Im Allgemeinen ist festzustellen, dass die Beschichtung 15 mit zunehmender Anzahl von Einzelschichten eine erhöhte Langzeitstabilität aufweist. Die mehrschichtige Beschichtung 15 umfasst erfindungsgemäß einen Haftvermittler bzw. einen Primer, der unmittelbar auf die Netzstruktur 10 aufgebracht wird bzw. ist. Die Oberfläche der Netzstruktur 10 kann elektropoliert sein. Der Haftvermittler verbessert die Anhaftung der folgenden, biofunktionalen Schicht, die erfindungsgemäß Heparin oder Albumin umfasst. Die biofunktionale Schicht kann zusätzlich mit einer weiteren Schicht eines Vernetzers oder mit einer weiteren Schicht eines Haftvermittlers bzw. Primers bedeckt sein.

Die Netzstruktur 10 gemäß Fig. 3a bis 3c wurde unter denselben Testbedingungen für 60 min einem Volumenstrom von 150 ml/min menschlichen Blutes ausgesetzt, wobei das Blut auf eine Temperatur von 37°C erwärmt war. Im Vergleich zu der unbeschichteten Netzstruktur gemäß Fig. 2a bis 2c zeigt sich deutlich, dass die mit der Beschichtung 15 versehene Netzstruktur 10 keine Anlagerung von Blutbestandteilen bewirkte. Die Beschichtung 15 wirkt also einer solchen Anlagerung von Blutbestandteilen wirkungsvoll entgegen.

Die Beschichtung 15 ist vorzugsweise abriebfest, so dass die Netzstruktur 10 durch einen Katheter an den Behandlungsort geschoben werden kann, ohne dass sich die Beschichtung 15 vollständig abreibt. Insbesondere ist vorgesehen, dass die Netzstruktur 10 durch Zuführung an den Behandlungsort auf einen Transportdraht geladen ist, der längsverschieblich innerhalb eines Katheters angeordnet ist. Die komprimierte Netzstruktur 10 wird dann mit Hilfe des Transportdrahts durch den Katheter geschoben, wobei die Netzstruktur 10 die Innenfläche des Katheters berührt. Dabei ist die Beschichtung 15 vorzugsweise derart abriebfest, dass beim einmaligen Durchschieben der Netzstruktur 10 durch einen Katheter mit einer Länge von 155 cm bis 165 cm höchstens 5% der Masse der Beschichtung 15 abgerieben wird. Dabei weist der Katheter vorzugsweise einen Innendurchmesser von höchstens 0,7 mm auf, wenn der Katheter zur Zuführung einer medizinischen Vorrichtung genutzt wird, die einen Flow-Diverter bildet. Für medizinische Vorrichtungen, die als Stent ausgebildet sind, oder für kleine Flow-Diverter können Katheter eingesetzt werden, die einen Innendurchmesser von höchstens 0,5 mm, insbesondere höchstens 0,4 mm, aufweisen. Dabei ist die Beschichtung 15 der medizinischen Vorrichtung jeweils so abriebfest, dass sie beim Durchschieben der Netzstruktur 10 durch Katheter mit derartigen Innendurchmessern und mit der zuvor genannten Länge im Wesentlichen vollständig erhalten bleibt.

Vorzugsweise kann die Netzstruktur 10 mehrfach durch den Katheter geschoben werden, ohne dass es zu einer signifikanten Reduktion der Beschichtung 15 führt. Die Beschichtung kann so abriebfest sein, dass nach dreimaligem Durchführen der Netzstruktur 10 durch einen Katheter höchstens 40%, insbesondere höchstens 30%, insbesondere höchstens 20%, insbesondere höchstens 10%, der Masse der Beschichtung durch Abrieb entfernt werden. Dies gilt auch, wenn der Katheter beim Hindurchschieben der Netzstruktur 10 eine Krümmung mit einem Krümmungsradius von höchstens 5 mm aufweist, und einen Bogen mit einem Winkel von 180° bildet.

Im Übrigen ist vorgesehen, dass die Beschichtung 15 langzeitstabil ist. Die Beschichtung 15 löst sich bei Kontakt mit Körperflüssigkeiten, insbesondere bei Kontakt mit Blut, nicht auf. Zumindest ist vorgesehen, dass die Beschichtung 15 während eines 30-tägigen Kontakts mit einer Körperflüssigkeit, insbesondere Blut, höchstens 3%, insbesondere höchstens 1%, vorzugsweise 0%, ihrer Masse verliert.

Dies lässt sich objektiv durch die Verwendung von Ersatzflüssigkeiten, beispielsweise 0,9-prozentiger NaCI-Lösung oder Ringer-Lactat-Lösung, nachweisen. Es ist auch möglich, die Netzstruktur 10 mit der Beschichtung 15 über einen Zeitraum von einer Stunde einem stationären Blutfluss von 150 ml/min auszusetzen, wobei die Netzstruktur in einem Schlauch implantiert ist, der einen Innendurchmesser von höchstens 4,0 mm aufweist. Vorzugsweise wird ein Schlauch verwende, dessen Innendurchmesser 3,2 mm beträgt. Bei einem solchen Test, der auch über mehr als 60 min, beispielsweise 240 mm oder 360 min, durchgeführt werden kann, nimmt die Schichtdicke der Beschichtung vorzugsweise nicht wesentlich ab. Dabei kann die Schichtdicke beispielsweise durch Rasterkraft-Mikroskopie (AFM) ermittelt und nachgewiesen werden. Alternativ kann der Test auch über einen Zeitraum von 30 Tagen oder länger durchgeführt werden, wobei die Netzstruktur, implantiert in einem Schlauch mit einem Innendurchmesser von höchstens 4 mm, vorzugsweise 3,2 mm, einer Ersatzflüssigkeit, beispielsweise 0,9-prozentiger NaCI-Lösung, ausgesetzt wird. Auch bei diesem Test nimmt die Schichtdicke der Beschichtung 15 vorzugsweise nicht ab, was ebenfalls über AFM (atomic force microscopy) nachweisbar ist.

### Bezuaszeichenliste

- 10: Netzstruktur
- 11: Netzstrukturelement
- 12: Steg
- 13: Verbinder
- 14: Zelle
- 15: Beschichtung

## Patentansprüche

1. Medizinische Vorrichtung zur Behandlung von neurovaskulären Erkrankungen mit einer selbstexpandierbaren Netzstruktur (10), die zumindest abschnittsweise eine gekrümmte Wandung bildet und in einem radial komprimierten Zustand einen Querschnittsdurchmesser aufweist, der höchstens 1 mm beträgt, wobei die Netzstruktur (10) aus wenigstens einem Netzstrukturelement (11) gebildet ist, das eine entlang eines Durchmessers der Netzstruktur (10) messbare Höhe aufweist, die höchstens 75 µm beträgt, und wobei das Netzstrukturelement (11) durch einen Steg (12) oder einen Draht gebildet ist und eine antithrombogene Beschichtung (15) umfasst, die eine Schichtdicke zwischen 5 nm und 100 nm aufweist und mehrschichtig ausgebildet ist, wobei wenigstens eine erste Schicht Albumin oder Heparin und eine zweite Schicht einen Haftvermittler umfasst, wobei die zweite Schicht zwischen dem Netzstrukturelement (11) und der ersten Schicht angeordnet ist.

2. Medizinische Vorrichtung nach Anspruch 1,
**dadurch gekennzeichnet, dass**
die antithrombogene Beschichtung (15) eine Schichtdicke zwischen 5 nm und 50 nm, insbesondere zwischen 10 nm und 30 nm, insbesondere zwischen 15 nm und 25 nm, insbesondere 20 nm, aufweist.

3. Medizinische Vorrichtung nach Anspruch 1 oder 2,
**dadurch gekennzeichnet, dass**
die Netzstruktur (10) im expandierten Zustand einen Querschnittsdurchmesser von höchstens 6,5 mm, insbesondere höchstens 6 mm, insbesondere höchstens 5,5 mm, insbesondere höchstens 5 mm, insbesondere höchstens 4,5 mm, aufweist.

4. System mit einer medizinischen Vorrichtung nach einem der vorhergehenden Ansprüche und einem Transportdraht, wobei die medizinische Vorrichtung auf dem Transportdraht und in einem komprimierten Zustand längsverschieblich innerhalb eines Zuführschlauches (Introducer) angeordnet ist, der einen Innendurchmesser von höchstens 0,7 mm, insbesondere höchstens 0,5 mm, insbesondere höchstens 0,4 mm aufweist.

5. Set mit einem Katheter und einer medizinischen Vorrichtung oder einem System nach einem der vorhergehenden Ansprüche, wobei der Katheter einen Katheterschlauch aufweist, dessen Innendurchmesser höchstens 0,7 mm, insbesondere höchstens 0,5 mm, insbesondere höchstens 0,4 mm, und dessen Länge zwischen 130 cm und 170 cm, insbesondere 155 cm und 165 cm, insbesondere 160 mm, beträgt.

6. Verfahren zur Herstellung einer medizinischen Vorrichtung nach einem der Ansprüche 1 bis 3, bei dem die folgenden Schritte ausgeführt werden:
- Bereitstellen der selbstexpandierbaren Netzstruktur (10);
- Aufbringen eines Haftvermittlers auf das Netzstrukturelement (11);
- Aufbringen einer antihrombogenen Schicht auf den Haftvermittler.

## Claims

1. A medical device for the treatment of neurovascular diseases, with a self-expandable mesh structure (10) which forms a curved wall at least in sections and has a cross sectional diameter which is at most 1 mm in a radially compressed state, wherein the mesh structure (10) is formed from at least one mesh structure element (11) which has a height which can be measured along a diameter of the mesh structure (10) which is at most 75 µm, and wherein the mesh structure element (11) is formed by a web (12) or by a wire and comprises an antithrombogenic coating (15) which has a layer thickness of between 5 nm and 100 nm and is multi-layered in configuration, wherein at least one first layer comprises albumin or heparin and a second layer comprises a bonding agent, wherein the second layer is disposed between the mesh structure element (11) and the first layer.

2. The medical device as claimed in claim 1, **characterized in that**
the antithrombogenic coating (15) has a thickness of between 5 nm and 50 nm, in particular between 10 nm and 30 nm, in particular between 15 nm and 25 nm, in particular 20 nm.

3. The medical device as claimed in claim 1 or claim 2, **characterized in that**
in the expanded state, the mesh structure (10) has a cross sectional diameter of at most 6.5 mm, in particular at most 6 mm, in particular at most 5.5 mm, in particular at most 5 mm, in particular at most 4.5 mm.

4. A system with a medical device as claimed in one of the preceding claims and a transport wire, wherein the medical device is disposed on the transport wire and is disposed in a compressed state inside a feeding tube (introducer) so as to be longitudinally displaceable, the feeding tube having an internal diameter of at most 0.7 mm, in particular at most 0.5 mm, in particular at most 0.4 mm.

5. A set with a catheter and a medical device or a system as claimed in one of the preceding claims, wherein the catheter has a catheter tube the internal diameter of which is at most 0.7 mm, in particular at most 0.5 mm, in particular at most 0.4 mm, and the length of which is between 130 cm and 170 cm, in particular between 155 cm and 165 cm, in particular 160 cm.

6. A method for the production of a medical device as claimed in one of claims 1 to 3, in which the following steps are carried out:
- providing the self-expandable mesh structure (10);
- applying a bonding agent to the mesh structure element (11);
- applying an antithrombogenic layer to the bonding agent.

## Revendications

1. Dispositif médical destiné au traitement de maladies neurovasculaires, doté d'une structure réticulaire auto-expansible (10) qui constitue au moins par sections une paroi courbée et présente, en état radialement comprimé, un diamètre de section transversale qui est au maximum de 1 mm, la structure réticulaire (10) étant composée d'au moins un élément de structure réticulaire (11) qui présente une hauteur, mesurable le long d'un diamètre de la structure réticulaire (10), qui est d'au maximum 75 µm, et l'élément de structure réticulaire (11) étant constitué par une barrette (12) ou un fil, et comprenant un revêtement anti-thrombogène (15) qui présente une épaisseur de couche de 5 nm à 100 nm et est constitué de plusieurs couches, au moins une première couche comprenant de l'albumine ou de l'héparine et une seconde couche un promoteur d'adhérence, la seconde couche étant disposée entre l'élément de structure réticulaire (11) et la première couche.

2. Dispositif médical selon la revendication 1, **caractérisé en ce que**
le revêtement anti-thrombogène (15) présente une épaisseur de couche de 5 nm à 50 nm, en particulier de 10 nm à 30 nm, en particulier de 15 nm à 25 nm, en particulier 20 nm.

3. Dispositif médical selon la revendication 1 ou 2, **caractérisé en ce que**
la structure réticulaire (10) présente, en état dilaté, un diamètre de section transversale d'au maximum 6,5 mm, en particulier d'au maximum 6 mm, en particulier d'au maximum 5,5 mm, en particulier d'au maximum 5 mm, en particulier d'au maximum 4,5 mm.

4. Système comportant un dispositif médical selon une des revendications précédentes et un fil de transport, le dispositif médical étant disposé sur le fil de transport et déplaçable en longueur en état comprimé à l'intérieur d'un tuyau d'alimentation (introducteur) qui présente un diamètre intérieur d'au maximum 0,7 mm, en particulier d'au maximum 0,5 mm, en particulier d'au maximum 0,4 mm.

5. Kit comportant un cathéter et un dispositif médical ou un système selon une des revendications précédentes, le cathéter présentant un tuyau de cathéter dont le diamètre intérieur est d'au maximum 0,7 mm, en particulier d'au maximum 0,5 mm, en particulier d'au maximum 0,4 mm, et dont la longueur est de 130 cm à 170 cm, en particulier 155 cm à 165 cm, en particulier 160 mm.

6. Procédé de production d'un dispositif médical selon une des revendications 1 à 3 dans lequel les étapes suivantes sont exécutées :
- mise à disposition de la structure réticulaire auto-expansible (10) ;
- application d'un promoteur d'adhérence sur l'élément de structure réticulaire (11) ;
- application d'une couche anti-thrombogène sur le promoteur d'adhérence.
